# EUROPEAN PATENT SPECIFICATION

(11) **EP 2 872 074 B1**
(45) Date of publication and mention of the grant of the patent: **20.12.2017**
(21) Application number: 13817174.9
(22) Date of filing: 09.07.2013
(51) Int. Cl.: A61F 2/78, A61F 13/02, A61K 31/00, A61L 15/00, C09J 7/04

(54) **NOVEL MEDICAL COUNTERMEASURE FOR FIRST RESPONDER USE IN MASS CASUALTY THERMAL AND/OR RADIOLOGICAL BURN INJURY EVENT**
NEUARTIGE MEDIZINISCHE GEGENMASSNAHME ZUR ERSTRESPONDER-VERWENDUNG IN EINEM MASSENWÄRME- UND/ODER STRAHLUNGSBRANDWUNDENEREIGNIS
NOUVELLE CONTRE-MESURE MÉDICALE DESTINÉE À DES PREMIERS INTERVENANTS, EN CAS DE BRÛLURES THERMIQUES ET/OU RADIOLOGIQUES AFFECTANT UN TRÈS GRAND NOMBRE DE BRÛLÉS

(30) Priority: 10.07.2012 US 201261669961 P
(43) Date of publication of application: 20.05.2015
(73) Proprietor: Bio Med Sciences, Inc., Allentown, PA 18106 (US)
(72) Inventor: DILLON, Mark, E., Center Valley, PA 18034 (US)
(74) Representative: Bressel, Burkhard
(86) International application number: PCT/US2013/049727
(87) International publication number: WO 2014/011636

(56) References cited:
- EP-A2- 0 475 807
- EP-B1- 0 874 609
- WO-A1-01/49228
- WO-A1-2010/060094
- WO-A1-2012/037065
- WO-A2-2007/070801
- WO-A2-2009/004282
- US-A1- 2005 038 368
- US-A1- 2012 093 759

## Description

### BACKGROUND OF THE INVENTION

### 1. Field of the Invention

This invention relates to a novel wound dressing design. Particularly, this invention relates to a wound dressing which incorporates a unique set of features ideally suited for use by first responders in a mass casualty thermal and/or radiological burn injury event. A particular embodiment of this invention may be economically mass-produced, has an long or indefinite shelf life, requires no special storage conditions, is not temperature sensitive, can be supplied in rolls, can be easily applied by persons with little or no training, immediately restores skin barrier function, provides an antimicrobial effect, reduces pain, manages wound exudate, accommodates edema, is transparent so that wounds may be visualized (e.g., seen) without dressing removal, and will not complicate the wound condition when proper medical attention is delayed for significant periods of time.

### 2. Description of the Prior Art

In the field of woundcare there exist several general categories of commonly used dressings. Each type of dressing has its advantages and disadvantages, and is indicated for certain wound conditions and user preferences.

Some dressings aggressively adhere to the wound surface. For example, conventional gauze integrates into the wound as healing occurs and eschar forms on the wound surface. Other types of dressings are designed to adhere to the surrounding intact tissue around the wound site, but not directly to the wound. Examples of this type of dressing include polyurethane films coated with acrylic pressure sensitive adhesive. Other types of dressings are designed to be substantially nonadherent. Examples of this type include polyethylene oxide hydrogels, but also non-hydrogel materials such as that described in my U.S. Patent number 4,832,009, which is incorporated herein by reference. The latter example is a dressing made from an interpenetrating polymer network ("IPN") of polytetrafluoroethylene ("PTFE") and silicone, and is presently marketed by Bio Med Sciences, Inc. of Allentown, PA as Silon®.

Many commercially available dressings incorporate antimicrobial substances to reduce or prevent infections. Typical examples of such antimicrobials include various ionic forms of silver, drugs such as Polymyxin B Sulfate, Bacitracin Zinc, Neomycin, or combinations thereof. In each case the active ingredient is delivered to the wound and is depleted from the dressing over time.

There are a wide variety of wound types. The terms "first", "second" and "third" degree are often used to describe the extent of the injury, particularly for burn injuries. First degree wounds involve only the epidermis or outermost layer of skin. A mild to moderate sunburn is a good example; the surface of the skin is not breached, there is no bleeding and no chance of infection. A second degree, or partial thickness injury, extends through the epidermis and into the dermis. As long as part of the dermis remains, the epidermis will regenerate and the wound will spontaneously heal if proper conditions are maintained. Failure to maintain proper conditions results in delayed healing and may even cause a partial thickness wound to convert to full thickness via infection and/or integration of the dressing into the wound.

A full thickness, or third degree injury, extends entirely through the dermis to the subdermal tissue. These wounds will not spontaneously heal because dermal tissue is missing and cannot generate and support epidermal tissue. In such cases a tissue transplant is required by harvesting intact skin from a donor site or the application of a biosynthetic skin substitute. In the former case a partial thickness autograft is taken so that a portion of the dermal layer is transplanted but a portion remains behind, thereby allowing both sites to heal. In the latter case, modern technology has provided several alternatives to reduce the need for donor tissue. Such products may provide a manufactured dermal base or cultured epithelial surface, but each is biologically derived.

Such products, however, are not without their own drawbacks. Commercial products such as Integra® (Integra LifeSciences, Inc. , Plainsboro, NJ) and TransCyte® (Advanced Biohealing, Westport, CT) require a high degree of expertise to apply and manage during the healing process. Biobrane® (Smith & Nephew Company, London, UK) contains a nylon fabric that is woven from tri-filament threads and covalently bonded with collagen peptides from a porcine dermal collagen source. The multiple filaments provide a high exposure to the wound surface resulting in an increased adherence to the wound. Because of its adhesive nature, Biobrane in particular requires a high degree of skill and continuing care to avoid wound complications. Additionally, these products, like all materials containing biological components, are readily degradable and usually require special storage conditions such as refrigeration. This leads to inherently short shelf lives.

In a mass casualty thermal and/or radiologic burn injury event, such as those modeled by Bell & Dallas in their paper titled "Vulnerability of populations and the urban health care systems to nuclear weapon attack - examples from four American cities," many challenges would immediately arise in managing large numbers of patients, particularly in view of the complications of ionizing radiation occurring concurrently.

The first challenge is that burn treatment is a highly specialized form of medical care, which is the reason why designated "burn centers" exist all around the world. In the United States there are approximately 1,500 "burn beds", in about 100 specialized facilities to treat burn patients. Of those, approximately 1,000 are occupied at any particular time. Consequently the resources needed to treat a large number of burn patients, hundreds or perhaps thousands, would immediately and completely overwhelm existing capacity. Logistically, the only course of action would be to transport and admit patients to conventional, non-specialized facilities for interim care until proper burn treatment can be provided. Furthermore, local resources and infrastructure could be significantly compromised thereby delaying the ability to mobilize and transport patients. Victims could be waiting hours or even days in a crisis zone before trained medical personal are available. For burn patients this is a life threatening and critical issue. Skin barrier function must be immediately restored and infection must be prevented if there is any hope of stabilization and eventual survival.

According to Mosteller RD. Simplified calculation of body-surface area. N Engl J Med 1987;317:1098, the average adult male has a total body surface area of approximately 1.9 square meters. So ideally, a mass casualty burn dressing would be delivered in conveniently transported sterilized units of enough material to cover that amount of surface area.

WO 2010/060094 A1 describes an antimicrobial laminate construct, which comprises an antimicrobial layer and an adhesive layer, wherein the antimicrobial layer comprises at least one antimicrobial agent and a binder. The antimicrobial agent is reported to be silver or other active agents.

WO 2009/004282 A2 describes a medical dressing for the prevention and/or treatment of skin damage, which comprises a plastics film layer suitable for nonadherent and readily removable contact with the skin, and a backing layer. The plastics film layer includes an antimicrobial or other bioactive agent. The antimicrobial agent may be silver, iodine or complexes thereof. The backing layer is a layer of gauze, a thin web of nonwoven fabric, a thin layer of foam or a piece of woven or knitted textile fabric.

WO 01/49228 A1 describes a wound dressing consisting of a multilayered composite structure, wherein one of the layers may be a silicone-containing compound comprising a thin layer made from an interpenetrating polymer network ("IPN") of polytetrafluorethylene and silicone which is manufactured under the trademark Silon-TSR®. This layer is laminated to a polyurethane foam.

EP 0 475 807 A2 describes wound-covering materials comprising a support layer formed of a film of a biocompatible highly aqueous gel-forming substance, at least a part of an area to be contacted with the wound being coated with a water-repellant substance and a moisture permeation-controlling layer formed on the support layer on the side opposite to the area to be contacted with the wound, and optionally, the support layer and/or moisture permeation-controlling layer containing an antimicrobial agent. This former layer may include an antimicrobial agent, such as a sulfa drug, an antibiotic or the like. Further, silver can be introduced into the support layer.

The countermeasure required to stabilize patients in such a scenario must be a wound dressing that is easily stockpiled, transported, and applied by people with little or no training. Furthermore, the dressing must not complicate the wound by allowing infection or wound adherence/integration to occur. The dressing must also accommodate often copious amounts of fluid or exudate produced by such wounds. Importantly, the dressing must also be elastic and flexible to accommodate the dramatic edema or swelling that occurs after thermal burn injury; else the patient may suffer compartmental syndrome when the compression of nerves, blood vessels, and muscle leads to tissue death from lack of oxygenation. Ideally the dressing would also be transparent and not require changes, i.e. a single application would suffice.

### SUMMARY OF THE INVENTION

I have unexpectedly discovered that a carefully designed silicone-PTFE IPN in conjunction with a silane-based antimicrobial surface treatment provides a wound dressing ideally suited for use in a mass casualty thermal and/or radiological burn injury scenario. The antimicrobial surface treatment comprises 3-methoxysilylpropyldimethylactadecyl ammonium chloride as an antimicrobial substance which is chemically bonded to a silicone elastomer.

A method for manufacturing the wound dressing comprises: (a) providing a first layer of said thin film of the silicon-PTFE IPN material; (b) depositing a second layer of said silane-based antimicrobial surface treatment on said first layer, by passing said first layer through a coating assembly and coating the first layer with said second layer; and (c) forming said first layer coated with said second layer into usable shapes such as rolls or sheets.

A thin elastic film approximately 50 microns thick of silicone-PTFE IPN material supported by a paper carrier substrate was coated on one side with a tacky silicone formulation containing 5 percent by weight 3-methoxysilylpropyldimethyloctadecyl ammonium chloride. The material was then passed through a tunnel style oven and cured. The roll of coated IPN on substrate was then passed through a die cutting apparatus to create small slits, or fenestrations, in the film approximately 2.5 mm long and spaced approximately 1.5 cm apart. The film was then removed from the carrier substrate and cut to approximately 20 cm widths and rewound onto itself into rolls approximately 10 meters long.

### BRIEF DESCRIPTION OF THE DRAWINGS

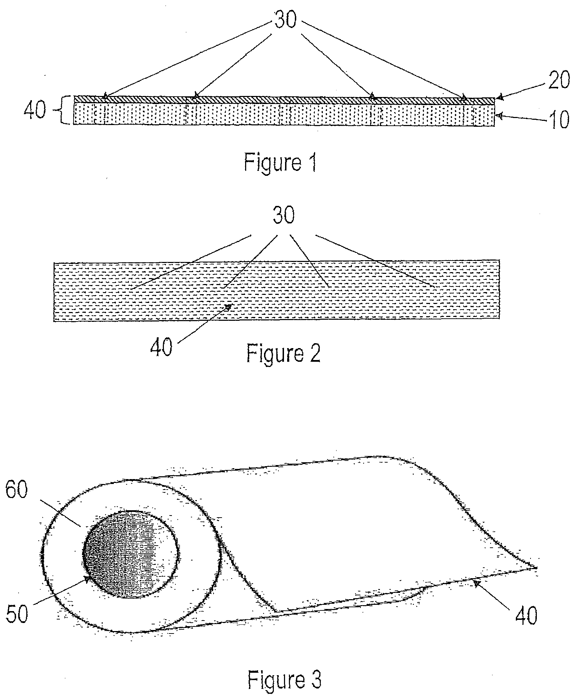

Figure 1 shows a cross-sectional view of a preferred embodiment of the inventive dressing (40), constructed in accordance with the invention, having a layer (10) of an IPN material that is coated with a layer (20) of tacky silicone containing 3-methoxysilylpropyldimethyloctadecyl ammonium chloride with fenestrations (30) cut in the dressing (40) at regular intervals.
Figure 2 is a top plan view of the silicone-PTFE IPN dressing (40) shown in Fig. 1, showing the fenestrations (30).
Figure 3 is a perspective view of the inventive dressing (40), constructed in accordance with the invention, wrapped onto a core (50) to form a roll (60) of the dressing (40).

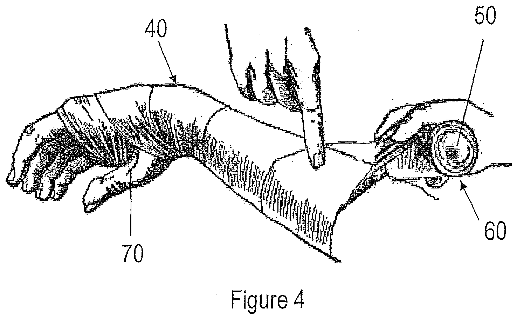

Figure 4 is a perspective view of the dressing (40) being applied to a thermal and/or radiological burn injury patient (70).

### DETAILED DESCRIPTION OF PREFERRED EMBODIMENTS

In a preferred embodiment of this invention, a wound dressing (40) comprises a 10 meter long sheet or layer (10) of a thin film of silicone-PTFE IPN material coated on one side with a coating (20) of a tacky silicone formulation containing 5 percent by weight 3-methoxysilylpropyldimethyloctadecyl ammonium chloride. Preferably, the wound dressing (40) is fenestrated with 2.5 mm slits (30) preferably separated by approximately 1.5 cm from each other, and the wound dressing (40) preferably is cut to be 20 cm wide, and preferably the wound dressing (40) is self-wound onto a plastic core (50) with the tacky coating (20) wrapped "in" against the core (50).

3-methoxysilylpropyldimethyloctadecyl ammonium chloride is particularly preferred because it readily bonds to a silicone-based substrate and it physically disrupts the cell membrane of the target organism (e.g., a germ) on contact. This means organisms do not metabolize the active agent and become resistant. Through extensive studies (including ISO 10993 standards), this colorless, non-leaching material was found to be safe and effective against a broad spectrum of fungi, bacteria, algae and yeast. Because 3-methoxysilylpropyldimethyloctadecyl ammonium chloride chemically bonds to a treated substrate of the invention, the dressing (40) itself becomes antimicrobial. This is an important difference from other commercially available antimicrobial dressings based on silver or other compounds which are delivered to the wound and are therefore depleted and lose effectiveness over time.

Both the Silon® dressing (that is, a Bio Med Sciences, Inc. dressing made from an interpenetrating polymer network ("IPN") of polytetrafluoroethylene ("PTFE") and silicone) and 3-methoxysilylpropyldimethyloctadecyl ammonium chloride materials are chemically stable, showing shelf life in the range of 5 years or more. Data to date actually suggests an indefinite shelf life.

The above preferred embodiment is not intended to be limiting, as variations on the illustrated design would be obvious to those skilled in the art. For example, precut sheets on a release liner may be used instead of rolls. Furthermore, it may be advantageous to use silver or other antimicrobial compounds, or combinations of such with 3-methoxysilylpropyldimethyloctadecyl ammonium chloride for clinically therapeutic reasons. Additionally, an antimicrobial agent may be incorporated into the non-wound contacting side of the dressing to reduce the overall bioburden of the site. By incorporating the antimicrobial agent into silicone-PTFE IPN material layer (10) microbes on the exterior surface of the wound site would be inhibited, thus improving the overall hygiene of the entire wound environment.

Lastly, the fenestration pattern itself may engineered to optimally accommodate edema by designing various cut-patterns to effect expansion characteristics or even provide built-in "break points" so that compartmental syndrome is avoided, although the elasticity of the IPN material of the inventive dressing (40) is expected to provide for sufficient expansion of the inventive dressing (40) to avoid compartmental syndrome. The following chart shows the remarkable elasticity of the basic silicone-PTFE IPN material without fenestrations.

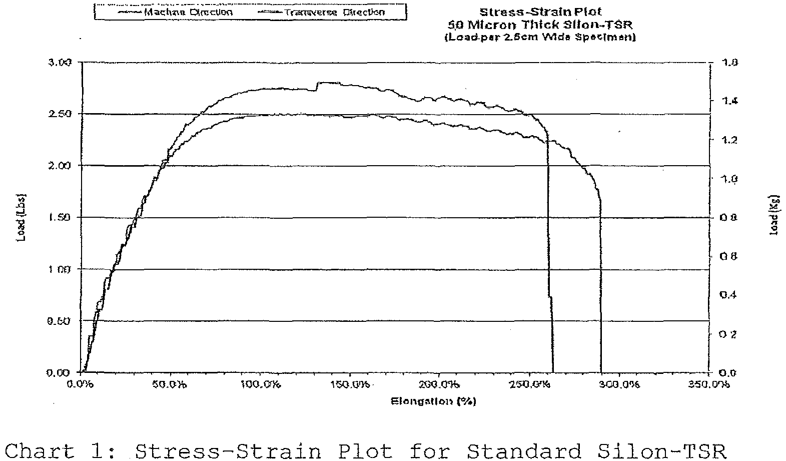

The following example is not intended to be limiting, as minor variations on the described processes would be obvious to those skilled in the art.

Likewise, it is believed that other materials could be used to achieve the same dressing design.

### Example 1:

A continuous sheet or layer (10), approximately 20 meters long and 40 cm wide, of silicone-PTFE IPN was manufactured according to established methods using a paper carrier substrate. The sheet or layer (10) of silicone-PTFE IPN film measured approximately 50 microns in thickness. The sheet or layer (10) of silicone-PTFE IPN film was then passed through a knife-over-roll assembly and coated with approximately 30 grams per square meter (gsm) of a silicone elastomer (product code 7-9600 from Dow Corning Corporation of Midland, MI), mixed with 5 percent by weight of 3-methoxysilylpropyldimethyloctadecyl ammonium chloride (product code HM4100 from BIOSAFE, Inc. of Pittsburgh, PA) to form a coating (20) of tacky silicone containing 3-methoxysilylpropyldimethyloctadecyl ammonium chloride on the sheet or layer (10) of the silicone-PTFE IPN film.

Using a rotary die cutting apparatus, fenestrations (30), preferably approximately 2.5 mm long, were preferably cut into the dressing (40). Reconfiguring the rotary die cutting apparatus for slitting and rewinding, the fenestrated dressing (40) was slit to 20 cm wide and rewound onto 2.5 cm diameter cores (50) in lengths of 10 meters with the coated side of the dressing (40) (that is, the side of the dressing (40) having the coating (20)) in contact with the plastic core (50).

When fenestrations (30) are not provided to the dressing (40), the same manufacturing process set out above for manufacturing fenestrated wound dressing (40) may be used to manufacture non-fenestrated wound dressing (40), except that the step of using the rotary die cutting apparatus to cut fenestrations into the wound dressing (40) may be skipped.

Samples of the product dressing (40) were tested using ASTM method E2149-01 - Shake for E. coli, with results showing a 3-log reduction in 2 hours, and a 4-log reduction in 24 hours. See Charts 2 and 3 below.

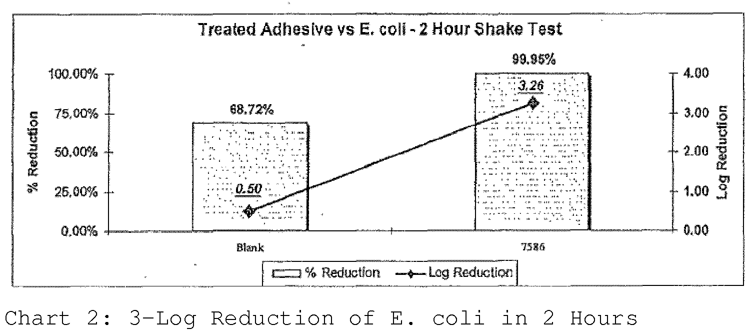

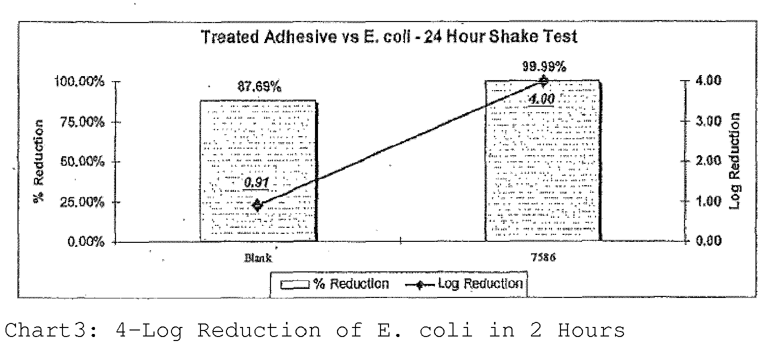

## Claims

1. A wound dressing (40) for treating a cutaneous injury, comprising a first layer (10) of thin film of a silicone-PTFE IPN material, and
a second layer (20) forming a silane-based antimicrobial surface treatment on the first layer (10) of the thin film of the silicone-PTFE IPN material,
the silane-based antimicrobial surface treatment comprising an antimicrobial substance chemically bonded to a silicone elastomer, the antimicrobial substance being 3-methoxysilylpropyldimethyloctadecyl ammonium chloride.

2. The wound dressing (40) of claim 1, wherein said first layer (10) has a first side; and wherein said second layer (20) is disposed on said first layer (10) first side, and wherein said second layer (20) on said first layer (10) first side forms a wound-contacting surface of said dressing (40).

3. The wound dressing (40) of any one of claims 1 or 2, wherein said antimicrobial substance is a substance that physically disrupts cell membranes of a target organism on contact therewith.

4. The wound dressing (40) of any one of claims 1 to 3, wherein said silicone elastomer mixed with the antimicrobial substance is adhered to said first layer (10) and provides an antimicrobial effect to the wound dressing (40); and wherein said wound dressing (40) has a long shelf life.

5. The wound dressing (40) of any one of claims 1 to 4, wherein said wound dressing (40) has a non-wound contacting side, and wherein an antimicrobial agent is incorporated into said non-wound contacting side of said wound dressing (40).

6. The wound dressing (40) of any one of claims 1 to 3, wherein said first layer (10) includes an antimicrobial agent.

7. The wound dressing (40) of any one of claims 1 to 3 and 6, wherein said second layer includes an antimicrobial agent in addition to the antimicrobial substance.

8. The wound dressing (40) of claim 7, wherein the antimicrobial agent is silver.

9. The wound dressing (40) of any one of claims 1 to 4, wherein an antimicrobial agent is incorporated into said wound dressing (40), and wherein said antimicrobial agent is silver.

10. The wound dressing (40) of any one of claims 1 to 9, wherein said wound dressing (40) comprises a plurality of fenestrations (30).

11. The wound dressing (40) of any one of claims 1 to 10, wherein the silicone elastomer contains 5 percent by weight of the antimicrobial substance.

12. A method of manufacturing the wound dressing (40) of any one of claims 1 to 11, comprising:
a) providing said first layer (10);
b) depositing said second layer (20) on said first layer (10), by passing said first layer (10) through a coating assembly and coating the first layer (10) with said second layer (20); and
c) forming said first layer coated with said second layer (20) into usable shapes such as rolls (60) or sheets.

13. The method of claim 12, wherein depositing said second layer (20) on said first layer (10) by passing said first layer (10) through a coating assembly comprises passing said first layer (10) through a knife-over-roll assembly and coating with a silicone elastomer containing 5% by weight of 3-methoxysilylpropyldimethyloctadecyl ammonium chloride.

## Patentansprüche

1. Wundverband (40) zum Behandeln einer kutanen Verletzung, umfassend eine erste Schicht (10) eines dünnen Films aus einem Silikon-PTFE-IPN-Material und
eine zweite Schicht (20), die eine silanbasierte antimikrobielle Oberflächenbehandlung auf der ersten Schicht (10) des dünnen Films aus dem Silikon-PTFE-IPN-Material ausbildet,
wobei die silanbasierte antimikrobielle Oberflächenbehandlung eine antimikrobielle Substanz umfasst, die chemisch an ein Silikonelastomer gebunden ist, wobei die antimikrobielle Substanz 3-Methoxysilylpropyldimethyloctadecyl-Ammoniumchlorid ist.

2. Wundverband (40) nach Anspruch 1, wobei die erste Schicht (10) eine erste Seite aufweist und wobei die zweite Schicht (20) auf der ersten Seite der ersten Schicht (10) angeordnet ist und wobei die sich auf der ersten Seite der ersten Schicht (10) befindende zweite Schicht (20) eine mit der Wunde in Kontakt kommende Oberfläche des Wundverbands (40) bildet.

3. Wundverband (40) nach einem der Ansprüche 1 oder 2, wobei die antimikrobielle Substanz eine Substanz ist, die die Zellmembranen eines Zielorganismus bei Kontakt mit diesem physikalisch zerstört.

4. Wundverband (40) nach einem der Ansprüche 1 bis 3, wobei das mit der antimikrobiellen Substanz vermischte Silikonelastomer mit der ersten Schicht (10) verbunden wird und dem Wundverband (40) einen antimikrobiellen Effekt verleiht und wobei der Wundverband (40) eine hohe Lagerbeständigkeit besitzt.

5. Wundverband (40) nach einem der Ansprüche 1 bis 4, wobei der Wundverband (40) eine nicht mit der Wunde in Kontakt kommende Seite aufweist und wobei ein antimikrobielles Mittel in die nicht mit der Wunde in Kontakt kommende Seite des Wundverbands (40) aufgenommen ist.

6. Wundverband (40) nach einem der Ansprüche 1 bis 3, wobei die erste Schicht (10) ein antimikrobielles Mittel umfasst.

7. Wundverband (40) nach einem der Ansprüche 1 bis 3 und 6, wobei die zweite Schicht zusätzlich zu der antimikrobiellen Substanz ein antimikrobielles Mittel umfasst.

8. Wundverband (40) nach Anspruch 7, wobei das antimikrobielle Mittel Silber ist.

9. Wundverband (40) nach einem der Ansprüche 1 bis 4, wobei ein antimikrobielles Mittel in den Wundverband (40) aufgenommen ist und wobei das antimikrobielle Mittel Silber ist.

10. Wundverband (40) nach einem der Ansprüche 1 bis 9, wobei der Wundverband (40) eine Vielzahl von Fensteranordnungen (30) umfasst.

11. Wundverband (40) nach einem der Ansprüche 1 bis 10, wobei das Silikonelastomer fünf Gewichtsprozent der antimikrobellen Substanz enthält.

12. Verfahren zur Herstellung des Wundverbands (40) nach einem der Ansprüche 1 bis 11, umfassend:
a) Bereitstellen der ersten Schicht (10),
b) Aufbringen der zweiten Schicht (20) auf die erste Schicht (10) durch Führen der ersten Schicht (10) durch eine Beschichtungsanordnung und Beschichten der ersten Schicht (10) mit der zweiten Schicht (20) und
c) Formen der mit der zweiten Schicht (20) beschichteten ersten Schicht (10) zu verwendbaren Gestaltungen, wie beispielsweise Rollen (60) oder Bahnen.

13. Verfahren nach Anspruch 12, wobei das Aufbringen der zweiten Schicht (20) auf die erste Schicht (10) durch Führen der ersten Schicht (10) durch eine Beschichtungsanordnung das Führen der ersten Schicht (10) durch eine Walzenspalt-Rakelanordnung und das Beschichten mit einem Silikonelastomer, das fünf Gewichtsprozent 3-Methoxysilylpropyldimethyloctadecyl-Ammoniumchlorid enthält, umfasst.

## Revendications

1. Pansement de plaie (40) pour traiter une blessure cutanée, comprenant une première couche (10) de film mince de matériau IPN de silicone-PTFE, et une seconde couche (20) formant un traitement de surface anti-microbien à base de silane sur la première couche (10) du film mince du matériau IPN de silicone-PTFE, le traitement de surface anti-microbien à base de silane comprenant une substance antimicrobienne liée chimiquement à un élastomère de silicone, la substance antimicrobienne étant le chlorure de 3-méthoxysilylpropyldiméthyloctadécyl ammonium.

2. Pansement de plaie (40) selon la revendication 1, dans lequel ladite première couche (10) a un premier côté; et dans lequel ladite seconde couche (20) est disposée sur le premier côté de ladite première couche (10), et dans lequel ladite seconde couche (20) sur le premier côté de ladite première couche (10) forme une surface de contact avec la plaie dudit pansement (40).

3. Pansement de plaie (40) selon l'une quelconque des revendications 1 ou 2, dans lequel ladite substance antimicrobienne est une substance qui rompt physiquement des membranes cellulaires d'un organisme cible lors d'un contact avec celle-ci.

4. Pansement de plaie (40) selon l'une quelconque des revendications 1 à 3, dans lequel ledit élastomère de silicone mélangé avec la substance antimicrobienne est mis à adhérer à ladite première couche (10) et procure un effet antimicrobien au pansement de plaie (40) ; et dans lequel ledit pansement de plaie (40) a une longue durée de conservation.

5. Pansement de plaie (40) selon l'une quelconque des revendications 1 à 4, dans lequel ledit pansement de plaie (40) a un côté de non-contact avec la plaie, et dans lequel un agent antimicrobien est incorporé dans ledit côté de non-contact avec la plaie dudit pansement de plaie (40).

6. Pansement de plaie (40) selon l'une quelconque des revendications 1 à 3, dans lequel ladite première couche (10) comporte un agent antimicrobien.

7. Pansement de plaie (40) selon l'une quelconque des revendications 1 à 3 et 6, dans lequel ladite seconde couche comporte un agent antimicrobien en plus de la substance antimicrobienne.

8. Pansement de plaie (40) selon la revendication 7, dans lequel l'agent antimicrobien est de l'argent.

9. Pansement de plaie (40) selon l'une quelconque des revendications 1 à 4, dans lequel un agent antimicrobien est incorporé dans ledit pansement de plaie (40) et dans lequel ledit agent antimicrobien est de l'argent.

10. Pansement de plaie (40) selon l'une quelconque des revendications 1 à 9, dans lequel ledit pansement de plaie (40) comprend une pluralité de fenestrations (30).

11. Pansement de plaie (40) selon l'une quelconque des revendications 1 à 10, dans lequel l'élastomère de silicone contient 5 pour cent en poids de la substance antimicrobienne.

12. Procédé de fabrication du pansement de plaie (40) de l'une quelconque des revendications 1 à 11, comprenant :
a) la fourniture de ladite première couche (10) ;
b) le dépôt de ladite seconde couche (20) sur ladite première couche (10), en faisant passer ladite première couche (10) à travers un ensemble d'enduction et en enduisant la première couche (10) de ladite seconde couche (20) ; et
c) la formation de ladite première couche enduite de ladite seconde couche (20) en formes utilisables telles que des rouleaux (60) ou des feuilles.

13. Procédé selon la revendication 12, dans lequel le dépôt de ladite seconde couche (20) sur ladite première couche (10) en faisant passer ladite première couche (10) à travers un ensemble d'enduction comprend le passage de ladite première couche (10) à travers un ensemble racle sur rouleaux et l'enduction d'un élastomère de silicone contenant 5 % en poids de chlorure de 3-méthoxysilylpropyldiméthyloctadécyl ammonium.
